# EUROPEAN PATENT SPECIFICATION

(11) **EP 3 088 808 B1**
(45) Date of publication and mention of the grant of the patent: **28.02.2018**
(21) Application number: 16167717.4
(22) Date of filing: 29.04.2016
(51) Int. Cl.: F24F 3/16, B01D 53/86, B03C 3/017, B60H 3/06, F24C 15/20

(54) **CLIMATE SYSTEM, AND BUILDING, VEHICLE OR COOKING FACILITY PROVIDED THEREWITH, AND METHOD FOR FILTERING AIR**
KLIMASYSTEM UND GEBÄUDE, FAHRZEUG ODER KOCHEINRICHTUNG DAMIT UND VERFAHREN ZUM FILTERN VON LUFT
SYSTÈME DE CLIMATISATION ET BÂTIMENT, VÉHICULE OU INSTALLATION DE CUISSON ÉQUIPÉ DE CELUI-CI, ET & xA;PROCÉDÉ DE FILTRATION DE L'AIR

(30) Priority: 01.05.2015 NL 2014750; 17.09.2015 NL 2015459
(43) Date of publication of application: 02.11.2016
(73) Proprietor: PlasmaMade B.V., 7951 SK Staphorst (NL)
(72) Inventor: van der Sluis, Martin Johan, 7951 PM Staphorst (NL)
(74) Representative: Verdijck, Gerardus

(56) References cited:
- WO-A1-2014/007626
- US-A- 5 368 816
- US-A1- 2013 183 214
- US-B1- 6 235 090

## Description

The present invention relates to a climate system. Such climate systems are for instance used in buildings, for instance in WTW systems, hospitals, production environments, cooking facilities, and so on.

Known climate systems make use of a fan to draw in air through a suction opening. The air is then conditioned in relation to temperature and/or humidity. The indrawn air is either discharged to the outside or recirculated.

WO2014/007626 A1 discloses a climate system with a filter system according to the preamble of claim 1. A problem with conventional climate systems is that they involve a number of components and/or require interaction with other systems. In addition, contaminations in the air are not sufficiently and/or efficiently removed.

The present invention has for its object to obviate or at least reduce one or more of the above problems with existing climate system.

The present invention provides for this purpose a climate system according to claim 1.

The filter system of the climate system according to the invention comprises an electrostatic filter and a so-called plasma filter.
The electrostatic filter preferably realizes a direct voltage of for instance 5 kV which is transmitted to a screen. A type of potential filter is hereby realized with which an electrostatic field is created. Contaminations, including particles such as smoke particles, fungi, odour particles and so on, can be captured by means of such a filter and thereby removed from the indrawn air.

Providing a plasma filter, with the plasma filter being capable of functioning as an ozone generator, achieves an additional air cleaning. The plasma filter generates ozone and/or radicals that react with contaminations, such as odours, in the indrawn air. Preferably, the ozone generator of the plasma filter is operatively connected to an ozone sensor. In practice, ozone is also referred to as plasma. For the purpose of this document ozone relates to ozone/plasma.

It has been found that in particular a combination of an electrostatic filter and a plasma filter achieves an effective cleaning due to the production and effective use of radical particles. These particles act on the cell walls and other parts of undesired particles/contaminations in the indrawn air.

An autonomous system is obtained by controlling the electrostatic filter and plasma filter with a controller using the measured characteristics of the indrawn air. These characteristics are determined with one or more sensors. The controller controls the fan with a drive. Preferably, the fan and the drive are both integrated in the housing of the climate system. Such an autonomous system provides the advantage that it can be operated independently. Application of the sensor according to the invention provides an optimal operation of the filter in relation to the actual extraction of air.

By providing the climate system with a sensor for measuring characteristics of the indrawn airflow, the control means, such as a process controller, receives information about the indrawn airflow. This information may include one or more of: air quantity, air speed, contamination including odours, etc. The controller may adjust frequencies and/or voltage levels of the electrostatic filter and/or plasma filter. This renders the climate system more effective as compared to conventional climate systems.

The one or more sensors can be provided in combination with the housing of the climate system. For example, the sensor can be attached to a grid or finishing/covering element or rosette. Also, one or more of the sensors can be provided in a room and/or building provided with the climate system according to the invention. This provides further information to the controller. The sensors may transmit and/or receive information to and/or from the controller with a wireless and/or wired protocol. This enables automatic climate control, for example enabling providing the most contaminated room in a building with the climate system according to the present invention with a filtered air flow.

Contaminations, particularly including odours, can be removed effectively from the indrawn air, thereby improving the climate in a building, room.

Furthermore, this filter control prevents unnecessary energy usage by over-treating the indrawn air. The controller may also control the drive of the fan, dependent on the type and amount of the contamination that is measured by a sensor in the indrawn air.

Preferably, the climate system is controlled on the basis of characteristics of the contaminations in the measured airflow, in particular including one or more of: smoke, pollen, bacteria, moisture, odours, viruses, fungi and so on. This information is provided to the controller that provides effective and efficient control of the climate system according to the invention.

In an advantageous preferred embodiment according to the present invention the filter comprises a carbon filter.

Providing a carbon filter or carbon mat achieves that excess ozone is captured. It is noted here that operation of the filter(s) is possible at a relatively low voltage with the filter according to the invention such that ozone production of the plasma filter is minimal.

An additional advantage of combining an electrostatic filter and carbon filter according to the invention is the increase in lifespan obtained for the carbon mat/filter as a result of an electric field being applied. A self-cleaning action of the filter according to the invention is hereby obtained. This renders the climate system according to the invention very effective. Also, the required maintenance and/or filter cleaning is significantly reduced, thereby rendering the system according to the invention effective.

In an advantageous preferred embodiment according to the present invention, the filter system comprises a glass fiber filter.

By providing a glass fiber filter, an improved filtering of contaminations out of the indrawn air is achieved. In particular, contaminations are trapped in the glass fiber filter. The glass fiber filter cooperates with the electrostatic filter such that an electric discharge eliminates the contaminations trapped in the glass fiber filter. This provides additional filtering of the indrawn air.

In an advantageous embodiment according to the present invention, the controller further comprises a user interface.

By providing a user interface an effective operation of the climate system is achieved. The user interface can be wireless and/or wired. The user interface renders the climate system flexible and adjustable to the specific conditions in which it is used. Preferably, the user interface comprises a touch screen, a tablet, mobile phone or other electronic device. By providing the user interface as a touch screen, tablet, mobile phone or similar electronic device, a user has easy access to the operating parameters of the climate system. This enables the user to control and/or adjust the filtering operation in an effective manner. For example, a so-called app can be downloaded to enable control via the electronic device of a specific climate system. Furthermore, this enable remote access for installing a software update, maintenance and the like. Also, the system can optionally send a warning signal to the user thereby achieving an interactive system.

In a further advantageous preferred embodiment according to the present invention a conical body is provided in or close to the suction opening.

By providing a conical body, the air drawn in using the fan is guided as optimally as possible through the electric filter. The surface area of the whole filter is hereby also utilized as fully as possible.

The conical body preferably provided with a shape such that a substantially uniform air distribution through the filter is realized during use. This further increases the effectiveness of the system.

The conical body is preferably provided with a number of ribs for better guiding of the airflow thereby. It has been found here that it is advantageous to provide the ribs in a spiral shape so as to thereby further improve the guiding of the airflow.

In the system according to a preferred embodiment of the invention, air with contamination enters a pre-chamber along with the indrawn contaminated air. The conical body preferably guides the air to the filter parts. Formed in a second chamber are radical particles with which the cleaning reactions take place substantially in a third chamber between the electrodes, preferably at the position of a glass fibre (nano)mat, which holds the particles. Following the reactions, discharge preferably takes place via an active carbon mat with which excess ozone is also captured. The outer casing of the system is shaped such that a further sound reduction is thereby realized.

Preferably, the housing comprises a plastic housing. A safer system is realized by providing a plastic housing when compared to a metal housing due to the insulating character of the housing. In addition, the overall weight of the filter system according to the invention is also reduced. This makes it easier to handle the system and assemble it on site.

The invention further also relates to a building, vehicle, kitchen facility and/or climate module such as an extractor hood or ceiling module provided with a climate system as described above.

Such a building, vehicle, kitchen facility and/or climate module such as an extractor hood and/or ceiling module provides the same effects and advantages as described for the filter system. Examples of vehicles are a car, plane, bus, and train. The system preferably comprises one or more sensors in rooms, compartments or sub-spaces of a building or vehicle.

In one of the preferred embodiments according to the invention a filter system according to the invention is preferably applied in extractor hoods in kitchens wherein airflow is extracted at a flow rate of about 1-1500 m³/hour. A plurality of extraction systems according to the invention can otherwise be employed within the same kitchen, and even within one extractor hood. It is also possible according to the invention to apply the filter in other extraction systems, for instance in so-called WTW systems. In addition to kitchens and WTW systems it is possible here to envisage application of the climate and/or filter system in smoking areas, production environments and hospitals. A plurality of extraction systems can be provided here in series and/or parallel. The filter system is preferably placed above the suction opening of the motor of for instance an extractor hood.

The invention further also relates to a method for filtering air according to claim 13. The method according to the invention provides the same advantages and effects as described for the climate system, building, vehicle, kitchen facility, such as an extractor hood.

Preferably, the method further comprises the step of providing user input with a user interface. This enables flexible operation of a climate system. The user interface preferably comprises a touch screen, tablet, mobile phone or other electronic device. This enables user friendly adjustments of controller settings.

It has been found that a self-cleaning action of the filters is realized with the preferred combination of filters as described above. This preferably realized self-cleaning action has a positive effect on the lifespan of the system and/or the time interval between maintenance operations.

Further advantages, features and details of the invention are elucidated on the basis of a preferred embodiment thereof, wherein reference is made to the accompanying drawings, in which:
- Figure 1 shows a view of the climate system according to the invention that is applied to a building;
- Figures 2A and B show details of the system of Figure 1;
- Figure 3 shows a view of the climate system provided in a kitchen facility with an extractor hood;
- Figure 4 shows a view of the climate system in an open room such as in a vehicle;
- Figure 5 shows a building with a climate system; and
- Figure 6A-F shows a climate module for connection to a ceiling or wall.

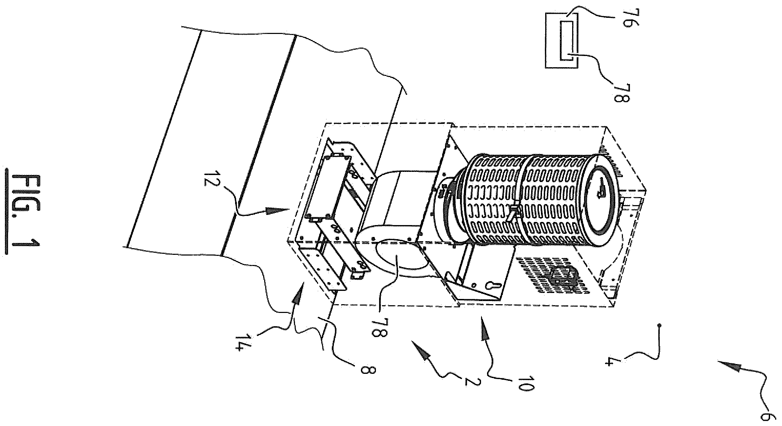

Climate system 2 (figure 1) is placed in room 4 of building 6. Climate system 2 comprises ducts 8 for moving air in building 6 from and to filter system 10. Filter system 10 has suction opening 12 that is connected to connection 14 of duct 8.

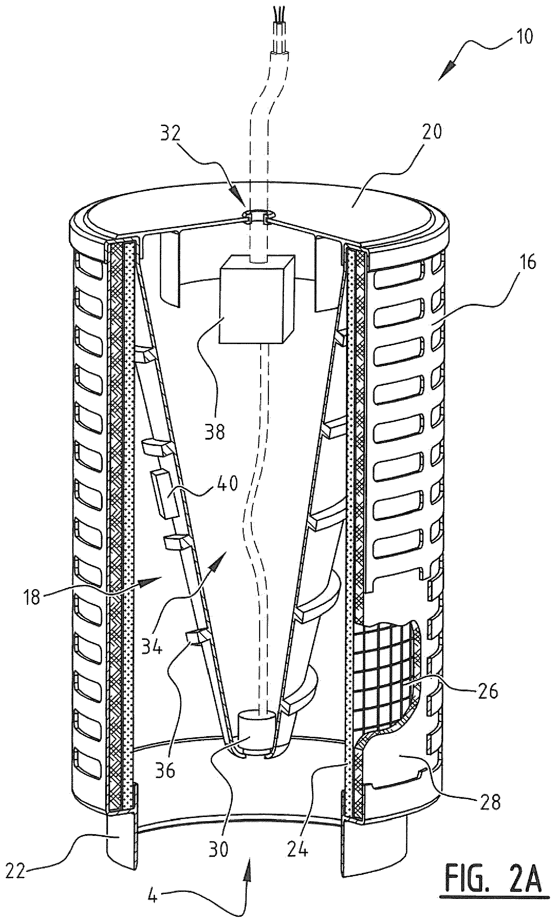

Filter system 10 (Figure 2A) is further provided with a plastic outer casing 16 and an inner cone 18 for guiding the indrawn air. Filter 10 is further provided with a plastic cover 20 and connecting flange 22 which in the shown embodiment has a diameter of about 150 mm. In the shown embodiment filter 10 is further provided with an inner pre-filter 24, an electrostatic grid 26 and an outer carbon filter 28.

In the shown embodiment grid 26 comprises a grid assembly with an inner and outer grid between which a glass fibre mat is arranged. Cover 20 is optionally provided with an open collector output, LEDs for status indication, and so on.

Filter 10 makes use of an airflow detector or sensor 30 which measures whether an airflow is present. Power supply to the electronics is provided via connection 32 which in the shown embodiment is a 12 V connection, preferably provided with an adapter for a 220 V or 110 V connection. Alternatively, the power supply is provided directly at 220 V. In the shown embodiment the conical body 18 is further provided with encapsulated electronics 34. The outer side of body 18 is provided with spiral-shaped ribs 36 for the purpose of improving the guiding of air. Also provided on the inner side in the shown embodiment is controller 38, which is connected to sensor 30 and activates or deactivates the electric filter.

In the shown embodiment Filter 10 is provided with a diameter of about 210 mm, with an inlet diameter of about 150 mm and a height of about 300 mm. An ozone generator 40 is also provided for an additional cleaning action.

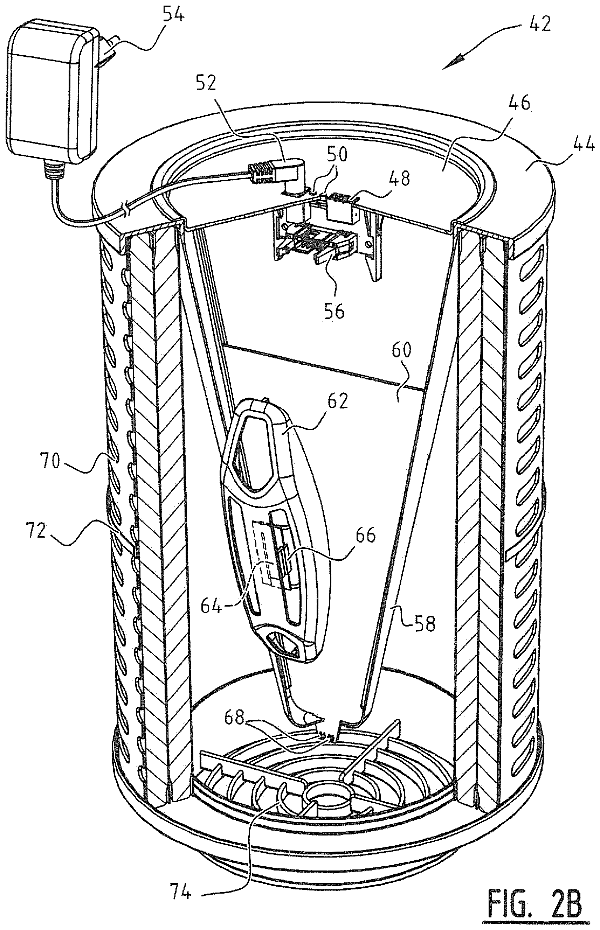

In an alternative embodiment, filter system 42 (figure 2B) is provided with an annular holder 44 on which cover 46 rests. Cover 46 is provided with connector 48, buttons 50 and a connection 52 for plug/adapter 54 which is connected to connector 56. Cone 58 is provided with printed circuit board 60 which is operatively connected to connector 56. Arranged on cone 58 is plasma generator 62 provided with ozone plate 64 which is arranged with clamping part 66 on generator 62. Printed circuit board 60 is provided with sensors 68 on the side directed downward during use. Outer casing 70 is provided with bracket 72 for mounting on for instance an extractor hood. The filter inlet is provided with grate 74.

Operation of system 2 (Figure 1) involves the use of control panel 76 with display 78.

When system 2 is activated, for instance with control 76, fan 78 will draw in air via opening 12. In the shown embodiment sensor 30 arranged on conical body 18 will detect the presence, and type and/or level of contamination, of the airflow. This detection signal is transmitted to controller 38 which in the shown embodiment is incorporated into the encapsulated electronics space 34. Controller 38 then activates ozone generator 40 and/or electrostatic filter, which is formed in the shown embodiment by the plastic outer casing 16, the inner pre-filter 24, the electrostatic grid 26, in combination with outer carbon filter 28. System 2 is hereby automatically activated using sensor 30 as soon as fan 78 becomes active.

In the shown embodiment of system 2 the voltage used preferably lies in the range of 0.05 Volt-10 kV, more preferably in the range of 0.5-2 kV in normal ambient conditions. A voltage of 2-4 kV is employed for the optionally provided ozone generator 40.

In the shown embodiment a standby mode is provided which can if desired be continuously "active".

It will be apparent that combinations with other types of filter are also possible. Filter system 2 can also be utilized in applications other than in a building 6.

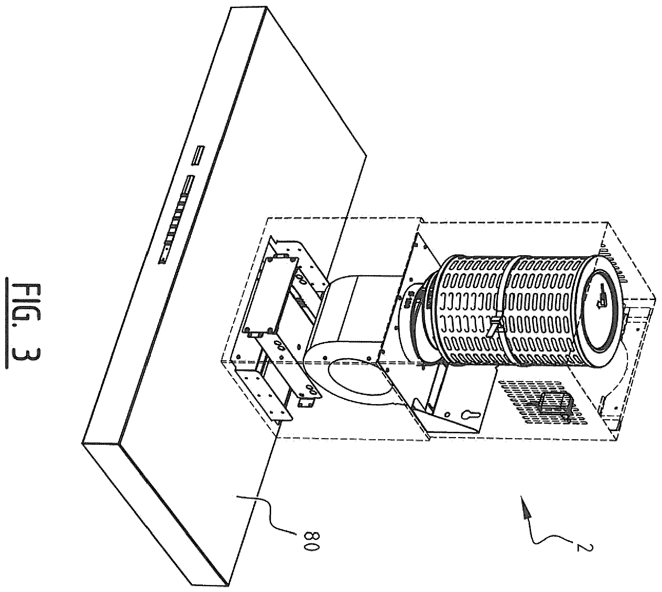

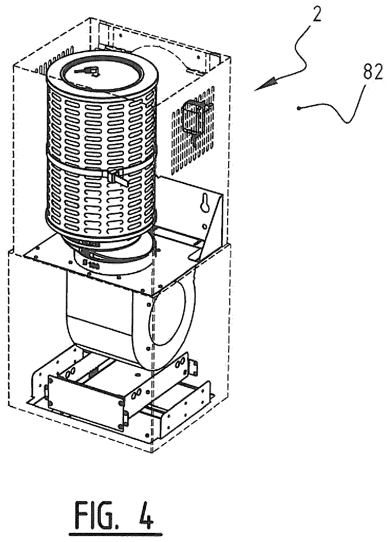

For example, climate system 2 (Figure 3) can be applied to kitchen facility, more specifically extractor hood 80. Alternatively, system 2 is provided in open room 82 (Figure 4). Open room 82 can be provided in a vehicle, airplane, building, etc.

Assembly of filter system 10 begins with a grating or mesh, i.e. grid 26, following which a second grating or mesh, i.e. grid 26, is provided at a short distance, for instance 40 mm, such that an inner and an outer annular cross-section are provided. The grids are connected. A glass fibre mat is preferably arranged in the intermediate space. Carbon mat 28 is arranged on the outer side of grid assembly 26. An ozone element 40 is subsequently arranged in a housing which is then placed on conical element 34. Conical element 34 can then be placed in the interior of grid assembly 26, wherein the wiring can be connected. An end plate 20 can be arranged following placing. In a currently preferred embodiment a printed circuit board is then adhered fixedly in filter 10. The end plate with sensor can subsequently be arranged so that filter 10 is mounted and can be applied.

Filter 10 has been tested in an experiment in a kitchen. Recirculation was started at about 200 m³/hour about 10 minutes prior to the cooking process in order to bring about a good circulation. In the applied situation the ozone binds the undesired particles with an efficiency of about 95%. The noise reduction realized was about 25% compared to the conventional extraction.

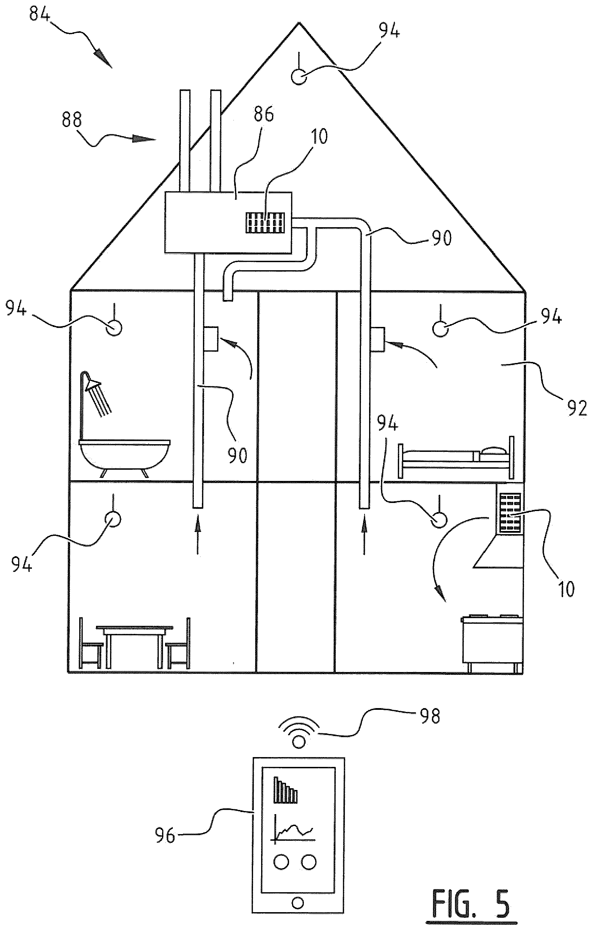

Building or house 84 (figure 5) comprises so-called heat recovery system ("WTW") 86 with filter 10. Inlets and/or outlets 88 are provided. Air ducts 90 are provided to relevant rooms 92 in house 84. Optionally, a kitchen can be provided with a separate filter 10. Sensors 94 provide climate information of room 92 to the controller of filter 10. Optionally, tablet 96 controls settings of filter 10 with an app using wireless signals 98, for example.

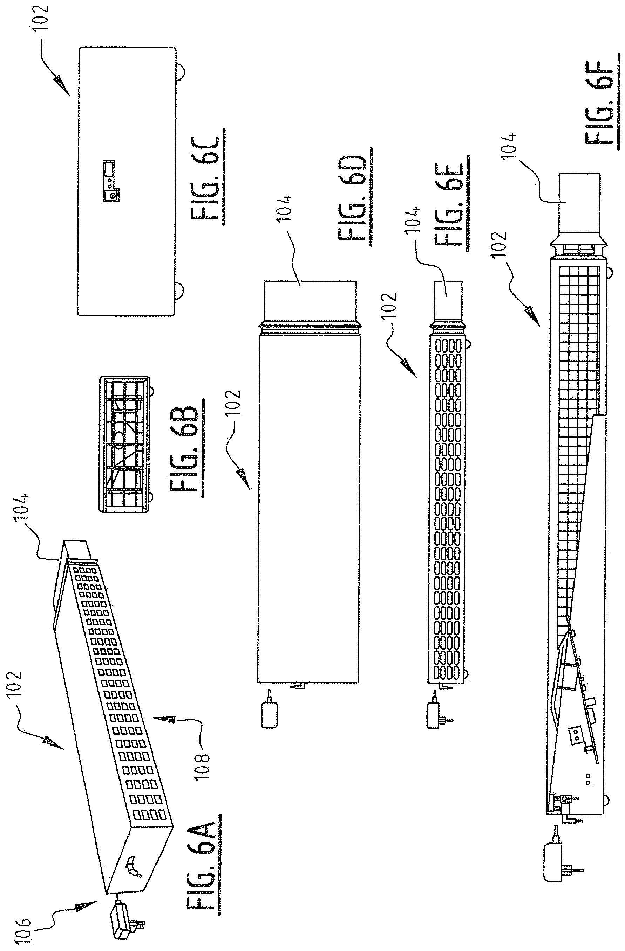

Climate module 102 (figure 6A-F) comprises an elastic connector 104 enabling flexible connection to other parts of a climate system. In the illustrated embodiment grid or grating 108 is provided on a side of module 102. Operational elements are similar to that of filter 10 are not described again in detail. Power is supplied by electrical connector 106.

## Claims

1. Climate system (2) with a filter system (10), comprising:
- a housing;
- a suction opening (12) that is provided in the housing;
- a fan (78) operatively connected to the suction opening (12) for drawing in air through the suction opening (12);
- a drive that is operatively connected to the fan for driving the fan;
- an electrostatic filter (26) configured for electrostatic filtering of the indrawn air;
- a plasma filter (62) configured for generating ozone and providing the ozone to the indrawn air;
**characterised by**
- a sensor (30) for measuring characteristics of the indrawn airflow; wherein the characteristics of the indrawn air at least include the amount and type of contaminations and
- control means operatively connected to the sensor (30) and configured for receiving information about the indrawn airflow and for providing autonomous operation of the climate system and controlling the electrostatic and plasma filters on the basis of the measured characteristics of the indrawn airflow and by controlling the drive that is operatively connected to the fan (78).

2. Climate system according to claim 1, further comprising a carbon filter (28).

3. Climate system according to claim 1 or 2, further comprising a glass fiber filter.

4. Climate system as claimed in claim 1, 2 or 3, wherein the controller further comprises a user interface.

5. Climate system according to claim 4, wherein the user interface comprises a touch screen, tablet or mobile phone.

6. Climate system according to one or more of the foregoing claims, wherein the controller is configured to adjust frequencies and/or voltage levels of the electrostatic filter and/or plasmafilter.

7. Climate system according to one or more of the foregoing claims, wherein a conical body is provided in or close to the suction opening.

8. Climate system as claimed in claim 7, wherein the conical body is provided with a shape such that a substantially uniform air distribution through the filter is realized during use.

9. Climate system as claimed in claim 7 or 8, wherein the conical body is provided with a number of ribs.

10. Climate system as claimed in claim 9, wherein the ribs provide a spiral shape.

11. Building or vehicle provided with a climate system as claimed in one or more of the foregoing claims, further comprising one or more sensors in rooms, compartments or sub-spaces.

12. Extractor hood comprising a climate system with a filter system as claimed in one or more of the foregoing claims.

13. Method for filtering air, comprising of:
- drawing in air through a suction opening (12) using a fan (78) and a drive, with the fan and the drive being integrated in a housing of a climate system (2) as claimed in one or more of the foregoing claims;
- filtering the indrawn air; and
- controlling the filtering with a controller based on measured characteristics of the indrawn air and controlling the drive that is operatively connected to the fan (78).

14. Method as claimed in claim13, further comprising the step of providing user input with a user interface.

15. Method as claimed in claim 13 or 14, wherein the filter system has a self-cleaning action during use.

## Patentansprüche

1. Klimasystem (2) mit einem Filtersystem (10), das Folgendes umfasst:
- ein Gehäuse;
- eine Ansaugöffnung (12), die in dem Gehäuse bereitgestellt ist;
- ein Gebläse (78), das mit der Ansaugöffnung (12) funktionsfähig verbunden ist, zum Einziehen von Luft durch die Ansaugöffnung (12);
- einen Antrieb, der mit dem Gebläse funktionsfähig verbunden ist, zum Antreiben des Gebläses;
- ein elektrostatisches Filter (26), das zum elektrostatischen Filtrieren der eingezogenen Luft eingerichtet ist;
- ein Plasmafilter (62), das zum Erzeugen von Ozon eingerichtet ist und die eingezogene Luft mit dem Ozon versieht;
**gekennzeichnet durch**
- einen Sensor (30) zum Messen von Eigenschaften des eingezogenen Luftstroms; wobei die Eigenschaften der eingezogenen Luft mindestens die Menge und Art an Verunreinigungen einschließen, und
- ein Steuerungsmittel, das funktionsfähig mit dem Sensor (30) verbunden ist und zum Empfangen von Informationen über den eingezogenen Luftstrom und zum Bereitstellen von autonomem Betrieb des Klimasystems und Steuern des elektrostatischen und des Plasmafilters auf der Basis der gemessenen Eigenschaften des eingezogenen Luftstroms und durch Steuern des Antriebs, der mit dem Gebläse (78) funktionsfähig verbunden ist, eingerichtet ist.

2. Klimasystem gemäß Anspruch 1, das weiter ein Kohlefilter (28) umfasst.

3. Klimasystem gemäß Anspruch 1 oder 2, das weiter ein Glasfaserfilter umfasst.

4. Klimasystem gemäß Anspruch 1,2 oder 3, wobei die Steuerung weiter eine Benutzerschnittstelle umfasst.

5. Klimasystem gemäß Anspruch 4, wobei die Benutzerschnittstelle einen berührungsempfindlichen Bildschirm, ein Tablet oder ein Mobiltelefon umfasst.

6. Klimasystem gemäß einem oder mehreren der vorhergehenden Ansprüche, wobei die Steuerung zum Einstellen von Frequenzen und/oder Spannungspegeln des elektrostatischen Filters und/oder Plasmafilters eingerichtet ist.

7. Klimasystem gemäß einem oder mehreren der vorhergehenden Ansprüche, wobei ein konischer Körper in oder nahe der Ansaugöffnung bereitgestellt ist.

8. Klimasystem gemäß Anspruch 7, wobei der konische Körper mit einer solchen Form bereitgestellt ist, dass eine im Wesentlichen gleichförmige Luftverteilung durch das Filter während der Benutzung realisiert wird.

9. Klimasystem gemäß Anspruch 7 oder 8, wobei der konische Körper mit einer Anzahl an Rippen versehen ist.

10. Klimasystem gemäß Anspruch 9, wobei die Rippen eine Spiralform bereitstellen.

11. Gebäude oder Fahrzeug, das mit einem Klimasystem gemäß einem oder mehreren der vorhergehenden Ansprüche versehen ist, das weiter einen oder mehrere Sensoren in Räumen, Abteilen oder Teilräumen umfasst.

12. Abzugshaube, die ein Klimasystem mit einem Filtersystem gemäß einem oder mehreren der vorhergehenden Ansprüche umfasst.

13. Verfahren zum Filtrieren von Luft, das Folgendes umfasst:
- Einziehen von Luft durch eine Ansaugöffnung (12) unter Verwendung eines Gebläses (78) und eines Antriebs, wobei das Gebläse und der Antrieb in einem Gehäuse eines Klimasystems (2) gemäß einem oder mehreren der vorhergehenden Ansprüche integriert sind;
- Filtrieren der eingezogenen Luft; und
- Steuern der Filtrierung mit einer Steuerung basierend auf gemessenen Eigenschaften der eingezogenen Luft und Steuern des Antriebs, der mit dem Gebläse (78) funktionsfähig verbunden ist.

14. Verfahren gemäß Anspruch 13, das weiter den Schritt des Bereitstellens einer Benutzereingabe mit einer Benutzerschnittstelle umfasst.

15. Verfahren gemäß Anspruch 13 oder 14, wobei das Filtersystem eine Selbstreinigungswirkung während der Benutzung aufweist.

## Revendications

1. Système de climatisation (2) avec un système de filtre (10), comprenant :
- une enceinte ;
- une ouverture d'aspiration (12) qui est disposée dans l'enceinte ;
- un ventilateur (78) relié à l'ouverture d'aspiration (12) pour aspirer l'air à travers l'ouverture d'aspiration (12) ;
- une commande qui est reliée au ventilateur pour entraîner le ventilateur ;
- un filtre électrostatique (26) configuré pour filtrer de manière électrostatique l'air aspiré ;
- un filtre à plasma (62) configuré pour générer de l'ozone et pour fournir l'ozone à l'air aspiré ;
**caractérisé par**
- un capteur (30) destiné à mesurer les caractéristiques du flux d'air aspiré ; dans lequel les caractéristiques de l'air aspiré comprennent au moins la quantité et le type de contaminations et
- un moyen de commande relié au capteur (30) et configuré pour recevoir des informations sur le flux d'air aspiré et pour assurer le fonctionnement autonome du système de climatisation et pour contrôler les filtres électrostatique et à plasma sur la base des caractéristiques mesurées du flux d'air aspiré et en contrôlant la commande qui est reliée au ventilateur (78).

2. Système de climatisation selon la revendication 1, comprenant en outre un filtre à charbon (28).

3. Système de climatisation selon la revendication 1 ou 2, comprenant en outre un filtre en fibre de verre.

4. Système de climatisation selon la revendication 1, 2 ou 3, dans lequel le dispositif de commande comprend en outre une interface utilisateur.

5. Système de climatisation selon la revendication 4, dans lequel l'interface utilisateur comprend un écran tactile, une tablette ou un téléphone mobile.

6. Système de climatisation selon l'une quelconque des revendications précédentes, dans lequel le dispositif de commande est configuré pour régler les fréquences et/ou les niveaux de tension du filtre électrostatique et/ou du filtre à plasma.

7. Système de climatisation selon l'une quelconque des revendications précédentes, dans lequel un corps conique est disposé dans ou près de l'ouverture d'aspiration.

8. Système de climatisation selon la revendication 7, dans lequel le corps conique est muni d'une forme telle qu'une distribution d'air sensiblement uniforme dans le filtre est réalisée pendant l'utilisation.

9. Système de climatisation selon la revendication 7 ou 8, dans lequel le corps conique est muni d'un nombre de nervures.

10. Système de climatisation selon la revendication 9, dans lequel les nervures offrent une forme de spirale.

11. Bâtiment ou véhicule muni d'un système de climatisation selon l'une quelconque des revendications précédentes, comprenant en outre un ou plusieurs capteurs dans des pièces, des compartiments ou des sous-espaces.

12. Hotte aspirante comprenant un système de climatisation équipé d'un système de filtre selon une ou plusieurs des revendications précédentes.

13. Procédé de filtrage de l'air, comprenant :
- l'aspiration de l'air dans une ouverture d'aspiration (12) à l'aide d'un ventilateur (78) et d'une commande, le ventilateur et la commande étant intégrés à une enceinte d'un système de climatisation (2) selon une ou plusieurs des revendications précédentes ;
- le filtrage de l'air aspiré ; et
- la commande du filtrage à l'aide d'un dispositif de commande sur la base de caractéristiques mesurées de l'air aspiré, et le contrôle de la commande qui est reliée de manière opérationnelle au ventilateur (78).

14. Procédé selon la revendication 13, comprenant en outre l'étape qui consiste à prévoir une entrée utilisateur avec une interface utilisateur.

15. Procédé selon la revendication 13 ou 14, dans lequel le système de filtre possède une action d'auto-nettoyage pendant l'utilisation.
